# EUROPÄISCHE PATENTANMELDUNG

(11) **EP 0 745 589 A1**
(43) Veröffentlichungstag der Anmeldung: **04.12.1996**
(21) Anmeldenummer: 96108043.9
(22) Anmeldetag: 21.05.1996
(51) Int. Cl.: C07D 207/26, C07D 201/08, B01J 23/656

(54) **Verfahren zur Herstellung von 2-Pyrrolidonen**

(30) Priorität: 02.06.1995 DE 19520258
(71) Anmelder: BAYER AG, 51368 Leverkusen (DE)
(72) Erfinder: Frohn. Lutz, Dr., 40699 Erkrath (DE); Jentsch, Jörg-Dietrich, Dr., 45468 Mülheim (DE); Zirngiebl, Eberhard, Dr., 51061 Köln (DE)

(57) **Zusammenfassung**

2-Pyrrolidone können durch simultane Umsetzung von Maleinsäure, Ammoniak oder einem primären Amin und Wasserstoff bei erhöhter Temperatur und erhöhtem Druck in flüssiger Phase an einem Trägerkatalysator hergestellt werden, wobei der Trägerkatalysator sowohl Palladium als auch Rhenium in metallischer oder gebundener Form enthält.

## Beschreibung

Die vorliegende Erfindung betrifft ein Verfahren zur Herstellung von 2-Pyrrolidonen durch simultane Umsetzung von Maleinsäureanhydrid, Ammoniak oder einem primären Amin und Wasserstoff bei erhöhter Temperatur und erhöhtem Druck in flüssiger Phase, wobei ein Trägerkatalysator, der sowohl Palladium als auch Rhenium in metallischer oder gebundener Form enthält, eingesetzt wird.

2-Pyrrolidon und insbesondere N-substituierte 2-Pyrrolidone sind wichtige polare Lösungs- und Extraktionsmittel, beispielsweise Lösungsmittel für Polymere, wie Polyurethane, Polyimide, Polyamide, Polyarylensulfide, und Extraktionsmittel, beispielsweise für Acetylen, Butadien, Aromaten. Solche Pyrrolidone sind weiterhin vielbenutzte Medien für chemische Umsetzungen (Ullmann, 4. Aufl., Bd. 19, Verlag Chemie 1980, S. 641-642).

Maleinsäureanhydrid (MSA) kann durch Hydrierung, etwa mit katalytisch angeregtem Wasserstoff, in Bernsteinsäureanhydrid übergeführt werden, das in Gegenwart von Wasser in die offenkettige Bernsteinsäure übergehen kann. MSA kann weiterhin durch Hydrierung direkt oder über die Stufe des Bernsteinsäureanhydrids in γ-Butyrolacton übergeführt werden, das in Gegenwart von Wasser in die offenkettige ω-Hydroxy-buttersäure übergehen kann. Weiterhin ist es möglich, das γ-Butyrolacton mit Hilfe von Ammoniak oder primären Aminen in 2-Pyrrolidon oder in N-substituierte 2-Pyrrolidone überzuführen. Weiterhin ist es möglich, MSA mit Ammoniak oder primären Aminen in Gegenwart von katalytisch angeregtem Wasserstoff direkt zu 2-Pyrrolidon oder N-substituierten 2-Pyrrolidonen umzusetzen.

Die genannten vielfältigen Reaktionsmöglichkeiten von MSA und seiner Folgeprodukte, die miteinander konkurrieren, lassen es wahrscheinlich sein, daß alle diese Produkte als Gemisch auftreten, wenn man MSA mit Ammoniak oder primären Aminen und katalytisch angeregtem Wasserstoff gleichzeitig umsetzt. Daher hat es nicht an Versuchen gefehlt, durch die Auswahl geeigneter Katalysatoren und durch die sukzessive Erreichung der einzelnen Reaktionsstufen, die sodann schrittweise weiter umgesetzt werden, eine möglichst hohe Selektivität bezüglich der gegebenenfalls N-substituierten 2-Pyrrolidone zu erreichen.

In CS 212 181 wird ein Herstellungsverfahren für N-Alkyl-2-pyrrolidone beschrieben, in welchem aus MSA bzw. aus Bernsteinsäureanhydrid und einem primären Amin zunächst das N-Alkyl-monoamid der Maleinsäure oder Bernsteinsäure hergestellt wird, welches an einem Hydrierkatalysator zum N-Alkylpyrrolidon cyclisiert wird. Als Hydrierkatalysatoren werden solche mit Elementen der I., II., V., VII. und VIII. Gruppe des Periodensystems (Mendelejew) genannt. Beispiele für solche Katalysatoren sind Cu-Zn, Cu-Zn-Cr, Raney-Ni, Raney-Co; als Beispiel für Edelmetall-Katalysatoren wird ein Mischkatalysator beschrieben, der aus 1 g Pt (0,2 Gew.-%)-Re (0,1 Gew.-%) auf γ-Al₂O₃, 6 g Raney-Cu und 3 g Raney-Co besteht.

Auf der Suche nach einem stabilen Katalysator wird in DE-OS 24 45 871 ein Verfahren zur Herstellung von 2-Pyrrolidon beschrieben, bei dem man MSA mit Ammoniak oder das Diammoniumsalz der Bernsteinsäure jeweils mit Wasserstoff umsetzt und hierbei einen Katalysator benutzt, der Ni und Re und/oder Mo enthält. Das Verhältnis zwischen Ni und Re bzw. Mo beträgt 1:0,001-0,25.

Ein weiteres Verfahren zur Herstellung von N-substituierten 2-Pyrrolidonen geht ebenfalls von Maleinsäureanhydrid, Maleinsäure und/oder Fumarsäure, einem primären Amin und Wasserstoff aus, die in Gegenwart eines Katalysators, der Co und mindestens eines der Elemente Mn, Cu, P, Mo und/oder Na enthält, umgesetzt werden (EP 460 474). Dieses Verfahren wird in Gegenwart eines Lösungsmittels bei erhöhter Temperatur und bei erhöhtem Druck durchgeführt.

Ein noch weiteres Verfahren zur Herstellung von N-substituierten 2-Pyrrolidonen geht ebenfalls aus von MSA bzw. Maleinsäure, die mit einem primären Amin in Gegenwart von katalytisch angeregtem Wasserstoff umgesetzt werden. Dieses Verfahren zeichnet sich weiterhin dadurch aus, daß das primäre Amin im Gemisch mit den zugehörigen sekundären und/oder tertiären Aminen eingesetzt werden kann und daß man in Gegenwart von Wasser und/oder Ammoniak arbeitet. Der eingesetzte Katalysator enthält mindestens ein Element der I., VII. oder VIII. Nebengruppe des Periodensystems (Mendelejew) und kann zusätzlich Elemente der VI. Nebengruppe enthalten. In den Ausführungsbeispielen werden als Katalysatoren benutzt: Cu-Al₂O₃ und Co-Cu-Mn-Mo-Na-H₃PO₄.

In US 4.800.227 wird die Herstellung von 2-Pyrrolidon oder seinen N-substituierten Derivaten durch Umsetzung von MSA mit Ammoniak oder primären Aminen und katalytisch angeregtem Wasserstoff beschrieben. Es werden Katalysatoren eingesetzt, die Palladium und auf separatem Träger wenigstens eines aus der Gruppe von Ruthenium, Rhodium und Rhenium enthalten. Die Ausführungsbeispiele zeigen die Anwendung eines Katalysatorgemisches, das aus Ru(5 %)/Al₂O₃ und Pd(5 %)/Al₂O₃ in pulvriger Form durch Mischen hergestellt worden ist. Diese beiden Katalysatorfraktionen werden durch einfaches Mischen in verschiedenen Verhältnissen zueinander eingesetzt. Das Verfahren wird in einem polaren flüssigen Reaktionsmedium, wie Wasser, Tetrahydrofuran oder Dioxan ausgeführt.

Die genannten Patentschriften zeigen die große Empfindlichkeit der zugrundeliegenden Reaktion gegenüber dem benutzten Katalysator. Aus den meisten Schriften geht hervor, daß unedle Katalysatoren, wie Raney-Ni, häufiger eingesetzt werden als Edelmetall-Katalysatoren; in Fällen, in denen sowohl Unedelmetall- als auch Edelmetall-Katalysatoren eingesetzt werden, stellt der Unedelmetall-Katalysator den übergroßen Anteil dar. Beim Einsatz von mehr als einem Edelmetall, etwa gemäß US 4.800.277, scheint es wesentlich zu sein, diese Metalle getrennt voneinander auf verschiedenen Trägerpartikeln einzusetzen.

Es wurde nun gefunden, daß ein Trägerkatalysator, der sowohl Pd als auch Re gemeinsam auf einem Träger enthält, besonders günstig in der Herstellung von gegebenenfalls am N-Atom substituierten 2-Pyrrolidonen ist. Hierbei ist es möglich, auf systemfremde Lösungsmittel beim Arbeiten in flüssiger Phase zu verzichten, wodurch besonders hohe Raum-Zeit-Ausbeuten erzielt werden können. Es wurde weiterhin gefunden, daß γ-Butyrolacton als mögliches Nebenprodukt nicht oder nur in untergeordnetem Maße auftritt. Weiterhin wurde gefunden, daß Tetrahydrofuran, welches durch Überhydrierung an Pd/Ru-Katalysatoren entsteht, im erfindungsgemäßen Maße ebenfalls nicht oder nur untergeordnet auftritt.

Die Erfindung betrifft ein Verfahren zur Herstellung von 2-Pyrrolidonen der Formel 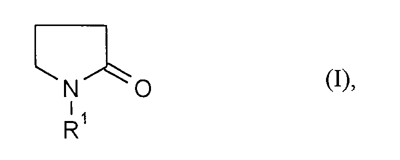 in der
- R¹: Wasserstoff, geradkettiges oder verzweigtes C₁-C₂₀-Alkyl, C₃-C₈-Cycloalkyl oder C₇-C₁₀-Aralkyl bedeutet,
das dadurch gekennzeichnet ist, daß man Maleinsäureanhydrid, ein primäres Amin der Formel

R¹-NH₂ (II),

worin
- R¹: die obigen Bedeutung hat,
und Wasserstoff simultan bei 150 bis 330°C und 10 bis 300 bar in flüssiger Phase diskontinuierlich oder kontinuierlich an einem Trägerkatalysator miteinander umsetzt, der sowohl Palladium als auch Rhenium in metallischer oder gebundener Form enthält, wobei Palladium in einer Menge von 0,5 bis 15 Gew.-% und Rhenium in einer Menge von 0,5 bis 10 Gew.-% vorliegen und die Summe beider Metalle 2 bis 15,5 Gew.-% beträgt, alles gerechnet als Metall und bezogen auf das Gesamtgewicht des Trägerkatalysators.

Geradkettiges oder verzweigtes C₁-C₂₀-Alkyl ist beispielsweise Methyl, Ethyl, Propyl, Isopropyl, Butyl, Isobutyl, tert.-Butyl, die isomeren Pentyle, Hexyle, Octyle, Decyle, Dodecyle, Tetradecyle, Hexadecyle, Octadecyle und Eicosyle.

C₃-C₈-Cycloalkyl ist beispielsweise Cyclopropyl, Cyclobutyl, Cyclopentyl, Cyclohexyl, Cycloheptyl, Cyclooctyl und (Poly)Methyl- sowie (Poly)Ethyl-Derivate von ihnen mit einer maximalen C-Atomzahl von 8.

C₇-C₁₀-Aralkyl ist beispielsweise Benzyl, α-Phenyl-ethyl, β-Phenyl-ethyl, Phenyl-propyl oder Phenyl-butyl.

In bevorzugter Weise werden primäre Amine der Formel

R¹¹-NH₂ (III)

umgesetzt, worin
- R¹¹: Wasserstoff geradkettiges oder verzweigtes C₁-C₁₀-Alkyl oder Benzyl bedeutet.

In besonders bevorzugter Weise werden primäre Amine der Formel

R²¹-NH₂ (IV)

umgesetzt, worin
- R²¹: Wasserstoff Methyl oder geradkettiges oder verzweigtes Octyl bedeutet.

Primäre Amine der genannten Art sind dem Fachmann bekannt und technisch in großen Mengen verfügbar. Im Sinne der Erfindung wird Ammoniak als primäres Amin angesehen (R¹ bzw. R¹¹ bzw. R²¹ = H).

Maleinsäureanhydrid (MSA) kann sowohl in reiner Form als auch in der Form, in der es technisch durch Oxidation von Butan, Buten oder Benzol gewonnen wird, eingesetzt werden.

Wasserstoff kann sowohl in reiner Form als auch in Form eines technisch verfügbaren Restgases, wie es etwa in petrochemischen Anlagen anfällt (H₂-Gehalt mindestens 70 Vol-%, Rest zu 100 % Methan, Ethan und andere bekannte Verunreinigungen), eingesetzt werden.

Das erfindungsgemäße Verfahren ist gekennzeichnet durch die Verwendung eines Trägerkatalysators, der sowohl Palladium als auch Rhenium in metallischer oder gebundener Form enthält. Als Träger kommen beispielsweise in Betracht: Kohle in verschiedenen Erscheinungsformen, SiO₂ oder Al₂O₃ in verschiedenen Erscheinungsformen, Alumosilikate, Zeolithe, Metalloxide, wie ZrO₂, TiO₂, ZnO, Bims, Phosphate und andere dem Fachmann bekannte Träger für Hydrierkatalysatoren.

Der erfindungsgemäß einzusetzende Katalysator enthält als Aktivbestandteile sowohl Palladium als auch Rhenium. Es hat sich herausgestellt, daß es wichtig ist, kein Gemisch verschiedener Katalysatoren, die einerseits Palladium und andererseits Rhenium enthalten, einzusetzen, sondern beide Aktivbestandteile gemeinsam auf einem Träger angeordnet zu haben. Die Menge an Palladium beträgt 0,5 bis 15 Gew.-%, bevorzugt 1 bis 12 Gew.-%; die Menge an Rhenium beträgt 0,5 bis 10 Gew.-%, bevorzugt 1 bis 8 Gew.-%; die Gesamtmenge beider Aktivbestandteile beträgt 2 bis 15,5 Gew.-%, bevorzugt 3 bis 13 Gew.-%. Alle Gewichtsangaben sind als Metall gerechnet und auf das Gesamtgewicht des Trägerkatalysators bezogen. Die Aktivbestandteile werden im allgemeinen in Form der Metallverbindungen auf den Träger gebracht, liegen aber beim Gebrauch des Katalysators offenbar überwiegend in metallischer Form vor, was zumindest auf das Palladium zutrifft. Es wird davon ausgegangen, ohne daß hierzu spezielle Untersuchungen vorliegen, daß das Rhenium in unmittelbarer räumlicher Nähe auf dem Katalysatorträger eine ausgeprägte aktivitäts- und selektivitätsfördernde Wirkung auf das Palladium ausübt. Diese aktivitäts- und selektivitätsfördernde Wirkung geht deutlich über die anderer Co-Katalysatoren hinaus, beispielsweise über die des Rutheniums, welches, insbesondere wenn es für sich separat auf einem Träger angeordnet ist, eine starke Neigung zur Überhydrierung zeigt.

Die Erfindung betrifft weiterhin den oben beschriebenen Katalysator.

Bevorzugte Träger sind Kohle, Al₂O₃, SiO₂ oder ZrO₂ in verschiedenen Erscheinungsformen. So kann Kohle sowohl als Graphit als auch als Aktivkohle in stückiger oder pulveriger Form eingesetzt werden. Kohle als Katalysatorträger kann innere Oberflächen von 50 bis 2000 m²/g aufweisen, A-Kohle im speziellen 400 bis 2000 m²/g. Al₂O₃ als Katalysatorträger hat im allgemeinen 50 bis 350 m²/g. SiO₂ als Katalysatorträger hat im allgemeinen 50 bis 600 m²/g. ZrO₂ als Katalysatorträger hat im allgemeinen 50 bis 200 m²/g. In besonders bevorzugter Weise wird Kohle, ganz besonders bevorzugt A-Kohle mit den genannten inneren Oberflächen eingesetzt.

Im allgemeinen kann sowohl Palladium als auch Rhenium im Überschuß gegenüber dem jeweils anderen Aktivbestandteil auf dem erfindungsgemäß einzusetzenden Katalysator vorliegen. Es ist jedoch bevorzugt, daß Palladium gegenüber Rhenium im Überschuß vorliegt. Hierzu wird ein Gewichtsverhältnis von Pd : Re = 1,5 bis 10:1, bevorzugt 2 bis 6:1 genannt.

Zur Herstellung des erfindungsgemäß einzusetzenden Katalysators kann man den Träger beispielsweise mit einer wäßrigen, gegebenenfalls sauren wäßrigen oder alkoholischen Lösung eines Pd-Salzes, beispielsweise PdCl₂, (NH₄)₂[PdCl₄], PdSO₄ oder Pd(NO₃)₂ tränken oder besprühen. Das Pd wird anschließend mit Hilfe von wäßrigem Alkalihydroxid (beispielsweise Natronlauge) als Oxid oder Hydroxid ausgefällt und so auf dem Träger fixiert. Anschließend wird der mit Pd ausgerüstete Träger weiterhin mit der wäßrigen oder alkoholischen Lösung einer Re-Verbindung getränkt oder besprüht, beispielsweise mit einer Lösung von ReCl₃, ReCl₄, ReO₂, ReO₃, Re₂O₇. Es hat sich als vorteilhaft erwiesen, das Re auf dem Träger dadurch zu fixieren, daß man das zum Aufbringen der Re-Verbindungen benutzte Lösungsmittel durch einfaches Trocknen entfernt. Im Anschluß an das Aufbringen der beiden Aktivbestandteile auf dem Träger können diese beispielsweise mit Wasserstoff, Kohlenmonoxid, Hydrazin oder Formaldehyd reduziert werden. Es ist jedoch gleichermaßen möglich, diese Reduktion im Verlaufe des erfindungsgemäßen Verfahrens ablaufen zu lassen. Hierbei geht mindest das Pd in den metallischen Zustand über.

Das erfindungsgemäße Verfahren wird in flüssiger Phase bei einer Temperatur von 150 bis 330, bevorzugt 200 bis 320°C, besonders bevorzugt 240 bis 300°C und erhöhtem Druck von 10 bis 300 bar, bevorzugt 50 bis 200 bar, besonders bevorzugt 70 bis 150 bar durchgeführt. Der erhöhte Druck ist im wesentlichen der des aufgedrückten H₂, beispielsweise in reiner Form oder in der oben beschriebenen mindestens 70 vol.-%igen Form; es kann aber auch zusätzlich ein Inertgas, wie Stickstoff oder ein inerter niedriger Kohlenwasserstoff, wie Methan oder Ethan, eingesetzt werden. Der Wasserstoff wird in jedem Falle in überschüssiger Menge eingesetzt, beispielsweise in der 2- bis 1000-fachen stöchiometrisch notwendigen Menge. MSA und das primäre Amin werden im molaren Verhältnis von 0,1 bis 10:1, bevorzugt 0,2 bis 5:1 eingesetzt. Bei diesem molaren Verhältnis werden vor allem wirtschaftliche Gesichtspunkte berücksichtigt: so wird die jeweils günstiger verfügbare Chemikalie im Überschuß eingesetzt, um die teurere Chemikalie möglichst weitgehend zu nutzen. Beispielsweise wird bei teuren primären Aminen das relativ billige MSA im Überschuß eingesetzt; nicht umgesetztes MSA kann zurückgewonnen und erneut in die Reaktion geführt werden. Andererseits kann das billige Ammoniak im Überschuß eingesetzt und nach der Reaktion zurückgewonnen werden.

Das erfindungsgemäße Verfahren kann diskontinuierlich oder kontinuierlich durchgeführt werden, beispielsweise in einem Autoklaven oder in einem als Verweilzeitstrecke dienenden druckfesten Rohraggregat.

Als Lösungsmittel können für den Fall ihrer Mitverwendung praktisch alle Lösungsmittel verwendet werden, die sich unter den Reaktionsbedingungen inert verhalten, z.B. Wasser, aliphatische und aromatische Kohlenwasserstoffe, wie Pentan, Hexan, Heptan, Octan, Benzol, Toluol oder Xylol, Ether, wie Diethylether, Diisopropylether, Methyl-tert.-butylether, Dioxan oder Tetrahydrofuran oder Gemische dieser Lösungsmittel. Vorteilhaft können auch N-substituierte und nicht N-substituierte Pyrrolidone, wie z.B. NMP und 2-Pyrrolidon, als Lösungsmittel benutzt werden.

### Beispiele

### Beispiel 1 (Katalysatorherstellung)

50 g pulverförmige Aktivkohle (Fa. Norit) wurden in 300 ml bidestilliertem Wasser aufgeschlämmt. Man setzte 13,5 g (60 mMol) Palladium-Acetat und 2,0 g (4,1 mMol) Re₂O₇ zu, ließ 24 h bei 20°C nachrühren und engte das Reaktionsgemisch bei 70°C und 10 mbar im Rotationsverdampfer zur Trockne ein. Nach 12 stündigem Trocknen bei 120°C im Trockenschrank erhielt man 62,6 g Katalysator, der 9,7 Gew.-% Pd, 2,3 Gew.-% Re und 2 Gew.-% Wasser enthielt.

### Beispiel 2 (Katalysatorherstellung)

20 g pulverförmiges γ-Al₂O₃ (Fa. Rhone-Poulenc) wurden in 120 ml bidestilliertem Wasser aufgeschlämmt. Man setzte 7,2 g (10,1 mMol) einer wäßrigen Na₂PdCl₄-Lösung zu, die 15 Gew.-% Pd enthielt, ließ 30 Minuten nachrühren und stellte mit 5 %iger NaOH auf pH 8. Man setzte 6 ml 20 %ige wäßrige Hydrazin-Lösung zu, ließ 30 Min. nachrühren und wusch den Kontakt auf der Nutsche chloridfrei. Der feuchte Filterkuchen wurde in 120 ml bidestilliertem Wasser aufgeschlämmt und mit 0,9 g Re₂O₇ (1,9 mMol), die in 5 ml bidestilliertem Wasser gelöst waren, versetzt. Man rührte 30 Minuten nach und engte das Reaktionsgemisch bei 70°C und 10 mbar im Rotationsverdampfer zur Trockne ein. Nach 12 stündigem Trocknen bei 120°C im Trockenschrank erhielt man 23,4 g Katalysator, der 5,4 Gew.-% Pd und 3,6 Gew.-Re enthielt.

### Beispiel 3

In einem Autoklaven wurden 49,5 g (0,5 mol) MSA, 100 g H₂O, 39 g wäßrige Methylaminlösung (40 Gew.-%) und 5 g Suspensionskatalysator (9,7 % Pd und 2,3 % Re auf Kohleträger) bei 275°C und 120 bar intensiv mit Wasserstoff kontaktiert. Nach 4 Stunden wurde der Versuch abgebrochen und der Autoklavinhalt gaschromatographisch analysiert. Die erzielte Ausbeute an NMP betrug 67 %.

### Beispiel 4

Wiederholung des Beispiel 3 mit 5 g Katalysator mit 5,4 % Pd und 3,6 % Re auf Al₂O₃ als Träger anstatt des in Beispiel 3 angegbebenen Katalysators. Die Ausbeute an NMP betrug 69 %.

### Beispiel 5

Wiederholung des Beispiels 3 mit 5 g Katalysator mit 9,7 % Pd und 2,3 % Re auf Al₂O₃ als Träger anstatt des in Beispiel 3 angegebenen Katalysators. Die Ausbeute an NMP betrug 62 %.

### Beispiel 6

Wiederholung des Beispiels 3 mit 5 g Katalysator mit 2 % Pd und 5 % Re auf Al₂O₃ als Träger anstatt des in Beispiel 3 angegebenen Katalysators. Die Ausbeute an NMP betrug 64 %.

### Beispiel 7

Wiederholung des Beispiels 3 mit 5 g Katalysator mit 5,4 % Pd und 3,6 % Re auf Al₂O₃ als Träger anstatt des in Beispiel 3 angegebenen Katalysators. Die Ausbeute an NMP betrug 68 %.

### Vergleichsbeispiel 1

Wiederholung des Beispiels 3 mit 5 g Katalysator mit 9,7 % Pd auf Kohleträger anstatt des in Beispiel 3 angegebenen Katalysators. Die Ausbeute an NMP betrug 25 %.

### Vergleichsbeispiel 2

Wiederholung des Beispiels 3 mit 5 g Katalysator mit 2,3 % Re auf Kohleträger anstatt des in Beispiel 3 angegebenen Katalysators. Es konnte kein NMP nachgewiesen werden.

### Vergleichsbeispiel 3

Wiederholung des Beispiels 3 mit 2,5 g Katalysator mit 9,7 % Pd auf Kohleträger und weiteren 2,5 g Katalysator mit 2,3 % Re auf separatem Kohleträger anstatt des in Beispiel 3 angegebenen Katalysators. Die Ausbeute an NMP betrug 56 %.

## Patentansprüche

1. Verfahren zur Herstellung von 2-Pyrrolidonen der Formel 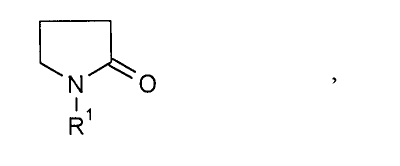 in der
R¹ Wasserstoff, geradkettiges oder verzweigtes C₁-C₂₀-Alkyl, C₃-C₈-Cycloalkyl oder C₇-C₁₀-Aralkyl bedeutet,
dadurch gekennzeichnet, daß man Maleinsäureanhydrid, ein primäres Amin der Formel
R¹-NH₂ ,
worin
R¹ die obigen Bedeutung hat,
und Wasserstoff simultan bei 150 bis 330°C und 10 bis 300 bar in flüssiger Phase diskontinuierlich oder kontinuierlich an einem Trägerkatalysator miteinander umsetzt, der sowohl Palladium als auch Rhenium in metallischer oder gebundener Form enthält, wobei Palladium in einer Menge von 0,5 bis 15 Gew.-% und Rhenium in einer Menge von 0,5 bis 10 Gew.-% vorliegen und die Summe beider Metalle 2 bis 15,5 Gew.-% beträgt, alles gerechnet als Metall und bezogen auf das Gesamtgewicht des Trägerkatalysators.

2. Verfahren nach Anspruch 1, dadurch gekennzeichnet, daß ein primäres Amin der Formel
R¹¹-NH₂
umgesetzt wird, worin
R¹¹ Wasserstoff geradkettiges oder verzweigtes C₁-C₁₀-Alkyl, Cyclopropyl, Cyclopentyl, Cyclohexyl oder Benzyl bedeutet.

3. Verfahren nach Anspruch 2, dadurch gekennzeichnet, daß ein primäres Amin der Formel
R²¹-NH₂
umgesetzt wird, worin
R²¹ Wasserstoff Methyl oder Octyl bedeutet.

4. Verfahren nach Anspruch 1, dadurch gekennzeichnet, daß bei 200 bis 320°C, bevorzugt bei 240 bis 300°C gearbeitet wird.

5. Verfahren nach Anspruch 1, dadurch gekennzeichnet, daß bei 50 bis 200 bar, bevorzugt bei 70 bis 150 bar gearbeitet wird.

6. Verfahren nach Anspruch 1, dadurch gekennzeichnet, daß Palladium in einer Menge von 1 bis 12 Gew.-% und Rhenium in einer Menge von 1 bis 8 Gew.-% vorliegen und die Summe beider Metalle 3 bis 13 Gew.-% beträgt, alles gerechnet als Metall und bezogen auf das Gesamtgewicht des Trägerkatalysators.

7. Verfahren nach Anspruch 1, dadurch gekennzeichnet, daß Palladium und Rhenium im Gewichtsverhältnis von Pd:Re = 1,5 bis 10:1, bevorzugt 2 bis 6:1 vorliegen.

8. Verfahren nach Anspruch 1, dadurch gekennzeichnet, daß als Träger Kohle, Al₂O₃, ZrO₂ oder SiO₂ eingesetzt wird.

9. Verfahren nach Anspruch 8, dadurch gekennzeichnet, daß als Träger Kohle mit einer inneren Oberfläche von 50 bis 2000 m²/g, bevorzugt A-Kohle mit einer inneren Oberfläche von 400 bis 2000 m²/g eingesetzt wird.

10. Katalysator, enthaltend 2 bis 15,5 Gew.-% Pd und Re auf einem gemeinsamen Träger für Hydrierkatalysatoren, wobei der Anteil an Pd 0,5 bis 15 Gew.-% und der Anteil an Re 0,5 - 10 Gew.-% betragen kann und alle Prozentangaben auf das Gesamtgewicht des Katalysators bezogen sind.
